(19) Europäisches Patentamt / European Patent Office / Office européen des brevets

(11) **EP 3 849 419 B1**

(12) **EUROPÄISCHE PATENTSCHRIFT**

(45) Veröffentlichungstag und Bekanntmachung des Hinweises auf die Patenterteilung:
**23.03.2022  Patentblatt 2022/12**

(21) Anmeldenummer: **20728934.9**

(22) Anmeldetag: **09.04.2020**

(51) Internationale Patentklassifikation (IPC):
**A61B 5/145** (2006.01)     **A61B 5/00** (2006.01)

(52) Gemeinsame Patentklassifikation (CPC):
**A61B 5/1451; A61B 5/14532; A61B 5/7203; A61B 5/725**

(86) Internationale Anmeldenummer:
**PCT/DE2020/200027**

(87) Internationale Veröffentlichungsnummer:
**WO 2020/211910 (22.10.2020 Gazette 2020/43)**

(54) **VERFAHREN UND VORRICHTUNG ZUM BESTIMMEN EINES AKTUELLEN GLUKOSEWERTS IN EINEM TRANSPORTFLUID**

METHOD AND DEVICE FOR DETERMINING A CURRENT GLUCOSE VALUE IN A TRANSPORTED FLUID

MÉTHODE ET DISPOSITIF POUR DÉTERMINER UNE VALEUR DE GLUCOSE ACTUELLE DANS UN FLUIDE DE TRANSPORT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.04.2019   DE 102019205430**

(43) Veröffentlichungstag der Anmeldung:
**21.07.2021   Patentblatt 2021/29**

(73) Patentinhaber: **EyeSense GmbH
63762 Großostheim (DE)**

(72) Erfinder:
• **KRUSE, Theresa
89073 Ulm (DE)**
• **GRAICHEN, Knut
89134 Blaubeuren (DE)**
• **MUELLER, Achim
63762 Großostheim (DE)**
• **KRIVÁNEK, Roland
63762 Großostheim (DE)**

(74) Vertreter: **Ullrich & Naumann PartG mbB
Schneidmühlstrasse 21
69115 Heidelberg (DE)**

(56) Entgegenhaltungen:
**WO-A1-2018/009614     US-A1- 2011 237 917**

**US-A1- 2013 079 613**

• **KERSTIN REBRIN ET AL: "Subcutaneous glucose predicts plasma glucose independent of insulin: implications for continuous monitoring", AMERICAN JOURNAL OF PHYSIOLOGY: ENDOCRINOLOGY AND METABOLISM., Bd. 277, Nr. 3, 1. September 1999 (1999-09-01), Seiten E561-E571, XP055714857, US ISSN: 0193-1849, DOI: 10.1152/ajpendo.1999.277.3.E561**
• **SCHALLER STEPHAN ET AL: "Robust PBPK/PD-Based Model Predictive Control of Blood Glucose", IEEE TRANSACTIONS ON BIOMEDICAL ENGINEERING, IEEE SERVICE CENTER, PISCATAWAY, NJ, USA, Bd. 63, Nr. 7, 1. Juli 2016 (2016-07-01), Seiten 1492-1504, XP011614178, ISSN: 0018-9294, DOI: 10.1109/TBME.2015.2497273 [gefunden am 2016-06-16]**
• **KOLLE KONSTANZE ET AL: "Meal detection based on non-individualized moving horizon estimation and classification", 2017 IEEE CONFERENCE ON CONTROL TECHNOLOGY AND APPLICATIONS (CCTA), IEEE, 27. August 2017 (2017-08-27), Seiten 529-535, XP033162558, DOI: 10.1109/CCTA.2017.8062516 [gefunden am 2017-10-06]**

**Beschreibung**

Die Erfindung betrifft ein Verfahren zum insbesondere kontinuierlichen Bestimmen eines aktuellen Glukosewerts in einem Transportfluid, insbesondere Blut, eines Organismus.

Die Erfindung betrifft weiter eine Vorrichtung zum insbesondere kontinuierlichen Bestimmen eines aktuellen Glukosewerts in einem Transportfluid, insbesondere Blut, eines Organismus.

Die Erfindung betrifft darüber hinaus eine Auswertevorrichtung zum insbesondere kontinuierlichen Bestimmen eines aktuellen Glukosewerts in einem Transportfluid, insbesondere Blut, eines Organismus.

Die Erfindung betrifft weiter ein nichtflüchtiges, computerlesbares Medium zur Speicherung von Instruktionen, welche ausgeführt auf einen Computer, bewirken, dass ein Verfahren zum insbesondere kontinuierlichen Bestimmen eines aktuellen Glukosewerts in einem Transportfluid, insbesondere Blut, eines Organismus, durchgeführt wird.

Obwohl die vorliegende Erfindung allgemein auf beliebige Verfahren zum Bestimmen eines aktuellen Glukosewerts in einem Transportfluid anwendbar ist, wird die vorliegende Erfindung in Bezug auf die Blutglukosekonzentration in einem Organismus erläutert.

Zur Bestimmung einer Blut-Glukosekonzentration BG in einem Organismus, insbesondere bei Menschen, sind Systeme zur kontinuierlichen Glukoseüberwachung, auch CGM - Continuous Glucose Monitoring - genannt, bekannt geworden. Bei einem CGM-System wird typischerweise eine interstitielle Gewebe-Glukosekonzentration IG automatisiert, beispielsweise alle ein bis fünf Minuten, gemessen. Insbesondere Diabetespatienten profitieren von CGM-Systemen, da im Vergleich mit Selbst-Überwachungs-Verfahren - auch Self-Monitoring-Verfahren genannt - bei welchem der Patient selbst manuell vier- bis zehnmal am Tag den Blut-Glukosewert bestimmt, Messungen mit einer deutlich höheren Frequenz durchgeführt werden können. Dies ermöglicht automatisierte Auswertungen und Warnsignale an den Patienten, insbesondere auch während der Patient schläft, was kritische Gesundheitszustände von Patienten zu vermeiden hilft.

Bekannte CGM-Systeme basieren einerseits auf elektrochemischen Vorgängen. Ein derartiges CGM System ist beispielsweise in der WO 2006/017358 A1 beschrieben. Darüber hinaus sind optische CGM-Systeme bekannt geworden, beispielsweise aus der DE 10 2015 101 847 B4, bei welchen eine vom Glukosewert abhängige Fluoreszenz ausgenutzt wird und welche hiermit durch Verweis einbezogen wird. Beide Arten von CGM-Systemen messen eine interstitielle Gewebe-Glukosekonzentration.

Es ist weiterhin bekannt, dass die Gewebe-Glukosekonzentration oder interstitielle Glukosekonzentration IG von der Blut-Glukosekonzentration, im Folgenden mit BG abgekürzt, abweicht. Eine große Abweichung besteht insbesondere nach starken Einflüssen auf den Blut-Glukosewert, beispielsweise durch Nahrungs- oder Nährstoffaufnahme oder beim Zuführen von Insulin, wie in der Nicht-Patentliteratur Basu, Ananda et al. "Time lag of Glucose from intravascular to interstitial compartment in humans." (Diabetes (2013): DB-131132), beschrieben. Diese Abweichung wird durch einen Diffusionsprozess im das Blut umliegende Gewebe verursacht, so dass der IG-Wert zeitverzögert und gedämpft dem BG-Wert nachfolgt, was beispielsweise in der Nicht-Patentliteratur Rebrin, Kerstin et al. "Subcutaneous Glucose predicts plasma Glucose independent of insulin: implications for continuous monitoring" (American Journal of Physiology-Endocrinology and Metabolism 277.3 (1999): E561-E571), beschrieben ist.

Auf Grund der beschriebenen Dämpfung und Zeitverzögerung zwischen den beiden Glukose-Konzentrationen, einerseits in Blut BG, andererseits im umgebenden Gewebe IG, führt eine Kalibrierung des CGM-Systems anhand einer manuellen Bestimmung der Blutglukosekonzentration, indem beispielsweise ein Bluttropfen aus dem Finger extrahiert und mittels eines externen Messgerätes die Glukosekonzentration im Bluttropfen bestimmt wird, zu erheblichen Ungenauigkeiten.

Um eine genaue Kalibrierung des CGM-Systems zu erzielen, muss jedoch der zuvor beschriebene Unterschied zwischen Gewebeglukosekonzentration und Blutglukosekonzentration berücksichtigt oder zumindest abgeschätzt werden. Hierfür sind verschiedene Verfahren bekannt geworden. Aus der Nicht-Patentliteratur Keenan, D. Barry et al. "Delays in minimally invasive continuous Glucose monitoring devices: a review of current technology." (Journal of diabetes science and technology 3.5 (2009): 1207-1214) ist es bekannt geworden, ein zeitversetztes Glukosesignal zur Kalibrierung zu verwenden. Weiterhin ist es aus der Nicht-Patentliteratur Knobbe, Edward J. and Bruce Buckingham "The extended Kalman filter for continuous Glukose monitoring." (Diabetes technology & therapeutics 7.1 (2005): 15-27) bekannt geworden, die Dämpfung und Zeitverzögerung des Diffusionsprozesses von Glukose zwischen Blut und Gewebe mittels eines Kalman-Filters zu kompensieren.

Auch US 2011/237917 A1 zeigt ein Verfahren zur Kalibrierung von

Blutzuckerüberwachungssensoren. US 2013/079613 A1 zeigt eine Vorrichtung zum Schätzen des Blutzuckerzustands.

Eine Aufgabe der vorliegenden Erfindung ist es daher, ein Verfahren, eine Vorrichtung sowie eine Auswertevorrichtung anzugeben, welche eine genauere Bestimmung des Glukosewerts insbesondere in Blut ermöglicht, bei gleichzeitiger höherer Flexibilität, insbesondere in Bezug auf die Berücksichtigung von weiteren Parametern und einfacherer Implementierung. Eine weitere Aufgabe der vorliegenden Erfindung ist es, ein alternatives Verfahren, eine alternative Vorrichtung sowie eine alternative Auswertevorrichtung anzugeben. Eine weitere Aufgabe der vorliegenden Erfindung ist es, ein Verfahren, eine Vorrichtung sowie eine Auswerteeinrichtung mit einer verbesserten Bestimmung der Blut-Glukosekonzentration in einem Organismus basierend auf einer Messung des interstitiellen Gewebe-Glukosewerts zur Verfügung zu stellen. Die Erfindung ist in den beigefügten Ansprüchen wiedergegeben.

In einer Ausführungsform löst die vorliegende Erfindung die vorstehend genannten Aufgaben durch ein Verfahren zum insbesondere kontinuierlichen Bestimmen eines aktuellen Glukosewerts in einem Transportfluid, insbesondere Blut, eines Organismus, umfassend die Schritte

a) Ermitteln, mittels einer Sensoreinrichtung, einer Messreihe, umfassend zumindest zwei zeitlich beabstandete Messwerte, für einen Gewebe-Glukosewert in dem das Transportfluid umgebenden Gewebe,

b) Ermitteln des Gewebe-Glukosewerts anhand der ermittelten Messreihe basierend auf einem Sensormodell, wobei mittels eines Sensormodells Messwerte der Sensoreinrichtung Gewebe-Glukosewerten unter Berücksichtigung von Messrauschen zugeordnet werden,

c) Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen, und

d) Ermitteln des aktuellen Glukosewerts basierend auf dem bereitgestellten Zustandsübergangsmodell und dem ermittelten Gewebe-Glukosewert,

wobei zumindest Schritt d), insbesondere die Schritte b)-d) unter Verwendung zumindest eines Moving Horizon-Schätzverfahrens durchgeführt wird.

In einer weiteren Ausführungsform löst die vorliegende Erfindung die vorstehend genannten Aufgaben durch eine Vorrichtung zum insbesondere kontinuierlichen Bestimmen eines aktuellen Glukosewerts in einem Transportfluid, insbesondere Blut, eines Organismus, vorzugsweise geeignet zur Durchführung eines Verfahrens gemäß einem der Ansprüche 1-12, umfassend

eine Sensoreinrichtung, insbesondere zur Messung von Fluoreszenz in einem das Transportfluid umgebenden Gewebe mittels einer Glasfasersonde, ausgebildet zum Ermitteln einer Messreihe, umfassend zumindest zwei zeitlich beabstandete Messwerte, für einen Gewebe-Glukosewert in dem das Transportfluid umgebenden Gewebe, eine Bereitstellungseinrichtung ausgebildet zum Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen, und zum Bereitstellen eines Sensormodells, wobei mittels eines Sensormodells Messwerte der Sensoreinrichtung Gewebe-Glukosewerten unter Berücksichtigung von Messrauschen zugeordnet werden, eine Auswerteeinrichtung ausgebildet zum Ermitteln des Gewebe-Glukosewerts anhand der ermittelten Messreihe basierend auf dem Sensormodell und zum Ermitteln des aktuellen Glukosewerts basierend auf dem bereitgestellten Zustandsübergangsmodell und dem ermittelten Gewebe-Glukosewert unter Verwendung zumindest eines Moving Horizon-Schätzverfahrens.

In einer weiteren Ausführungsform löst die vorliegende Erfindung die vorstehend genannten Aufgaben durch eine Vorrichtung zum insbesondere kontinuierlichen Bestimmen eines aktuellen Glukosewerts in einem Transportfluid, insbesondere Blut, eines Organismus, vorzugsweise geeignet zur Durchführung eines Verfahrens gemäß einem der Ansprüche 1-12, umfassend

zumindest eine Schnittstelle zum Anschluss einer Sensoreinrichtung zur Bereitstellung von einer Messreihe, umfassend zumindest zwei zeitlich beabstandete Messwerte, für einen Gewebe-Glukosewert in dem das Transportfluid umgebenden Gewebe, zumindest einen Speicher zum Speichern eines Zustandsübergangsmodells, wobei den mittels des Zustandsübergangsmodells ermittelten Gewebe-Glukosewerten zumindest ein Glukosewert in dem Transportfluid zugeordnet wird unter Berücksichtigung zumindest eines Prozessrauschwerts, und zum Speichern eines Sensormodells, wobei mittels des Sensormodells Messwerte der Sensoreinrichtung Gewebe-Glukosewerten unter Berücksichtigung von zumindest einem Messrauschwert zugeordnet werden, und eine Recheneinrichtung ausgebildet zum Ermitteln des Gewebe-Glukosewerts anhand der ermittelten Messreihe basierend auf dem gespeicherten Sensormodell und zum Ermitteln des aktuellen Glukosewerts basierend auf dem gespeicherten Zustandsübergangsmodell und dem ermittelten Gewebe-Glukosewert unter Verwendung zumindest eines Moving Horizon-Schätzverfahrens.

In einer weiteren Ausführungsform löst die vorliegende Erfindung die vorstehend genannten Aufgaben durch ein nichtflüchtiges, computerlesbares Medium zur Speicherung von Instruktionen, welche, ausgeführt auf einem Computer, bewirken, dass ein Verfahren zum insbesondere kontinuierlichen Bestimmen eines aktuellen Glukosewerts in einem Transportfluid, insbesondere Blut, eines Organismus, durchgeführt wird, vorzugsweise geeignet zur Durchführung eines Verfahrens gemäß einem der Ansprüche 1-12, umfassend die Schritte

a) Ermitteln, mittels einer Sensoreinrichtung, einer Messreihe, umfassend zumindest zwei zeitlich beabstandete Messwerte, für einen Gewebe-Glukosewert in dem das Transportfluid umgebenden Gewebe,
b) Ermitteln des Gewebe-Glukosewerts anhand der ermittelten Messreihe basierend auf einem Sensormodell, wobei mittels eines Sensormodells Messwerte der Sensoreinrichtung Gewebe-Glukosewerten unter Berücksichtigung von Messrauschen zugeordnet werden,
c) Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen, und
d) Ermitteln des aktuellen Glukosewerts basierend auf dem bereitgestellten Zustandsübergangsmodell und dem ermittelten Gewebe-Glukosewert,

wobei zumindest Schritt d), insbesondere die Schritte b)-d) unter Verwendung zumindest eines Moving Horizon-Schätzverfahrens durchgeführt wird.

Mit anderen Worten wird ein Verfahren zur Bestimmung einer Blutglukosekonzentration in einem Organismus vorgeschlagen. Dieses weist folgende Verfahrensschritte auf:

In einem ersten Verfahrensschritt erfolgt ein Aufnehmen einer Messreihe mit zumindest zwei zeitlich beabstandeten Sensormesswerten eines interstitiellen Gewebeglukosewerts des Gewebes des Organismus mittels eines Sensors. In einem weiteren Schritt erfolgt ein Bereitstellen eines Sensormodells des Zusammenhangs zwischen den Sensormesswerten und dem Gewebeglukosewert und ein Bereitstellen eines Zustandsübergangsmodells, welches ein Modell für den Zusammenhang zwischen Gewebeglukosewert und Blutglukosewert umfasst und in einem weiteren Verfahrensschritt erfolgt ein Quantifizieren des Blutglukosewertes des Organismus mittels des Sensormodells und des Zustandsübergangsmodells abhängig von den Sensor-Messwerten, wobei wesentlich ist, dass ein Moving-Horizon-Schätzverfahren verwendet wird.

Das Moving Horizon-Schätzverfahren, kurz MHE, ist an sich bei der Auswertung von Messsignalen, welche als Zeitreihen vorliegen, bekannt. Untersuchungen der Anmelderin haben ergeben, dass im Gegensatz zu den bisher verwendeten Verfahren die Verwendung eines Moving Horizon-Schätzverfahrens erhebliche Vorteile bei der Auswertung der Sensor-Messwerte zur Quantifizierung der Blutglukosewerte bietet, sowohl im Hinblick auf die Genauigkeit der Schätzung, als auch hinsichtlich der Flexibilität bezüglich der Annahmen für die Modelle als auch bezüglich der Schnelligkeit bei der Bereitstellung der Blutglukosewerte. Bevorzugt führt das Moving Horizon-Schätzverfahren zu einer aktuellen Blutglukosekonzentration und einer retrospektiven Blutglukosekonzentration, wobei die retrospektive Blutglukosekonzentration vorzugsweise unter Berücksichtigung zumindest einer vergangenen Blutglukosekonzentration (einer zuvor bestimmten Blutglukosekonzentration über den Werteverlauf (Konzentrationsverlauf)) bestimmt wird. Die retrospektive Blutglukosekonzentration ermöglicht hierbei insbesondere eine bessere Rekonstruktion des aktuellen Blutglukosemesssignals als lediglich die aktuelle Blutglukosekonzentration.

Die Auswerteeinrichtung kann dabei insbesondere ein Computer, ein integrierter Schaltkreis oder dergleichen sein, der insbesondere zur optimierten Berechnung, beispielsweise der Spur einer Matrix ausgebildet ist. Vorrichtung und/oder Auswerteeinrichtung können als tragbares Gerät mit unabhängigen Energiequellen, beispielsweise Akkus oder dergleichen ausgebildet sein, was einen effizienten Betrieb, mithin also den Energieverbrauch zur Durchführung des Verfahrens gemäß einer Ausführungsform der vorliegenden Erfindung möglichst geringhält, um einen möglichst langen Akkubetrieb zu ermöglichen, was die Nutzererfahrung verbessert. Hierzu sind insbesondere stromsparende Prozessoren, Schaltungen, Schaltkreise, Schnittstellen, insbesondere Drahtlosschnittstellen und dergleichen einsetzbar. Die Durchführung des Verfahrens kann dabei insbesondere hinsichtlich seiner Parameter, beispielsweise an die zugrundeliegende Vorrichtung beziehungsweise Auswerteeinrichtung angepasst werden, beispielsweise hinsichtlich des Auswertehorizonts und/oder des Rauschhorizonts, was im Folgenden beschrieben wird, um einerseits eine genügende Genauigkeit, andererseits eine lange Laufzeit zu erreichen.

Einer der damit erzielten Vorteile ist, dass damit eine zeit- und computerressourceneffiziente Schätzung des aktuellen Glukosewerts im Transportfluid, insbesondere Blut ermöglicht wird. Darüber hinaus ist ein Vorteil, dass die Flexibilität gegenüber bekannten Verfahren wesentlich erhöht wird, da Beschränkungen auf bestimmte Sensormodelle und/oder Zustandsübergangsmodelle entfallen. Ein weiterer Vorteil ist darüber hinaus, dass nicht nur die Genauigkeit des aktuellen Glukosewerts erhöht wird, sondern gleichzeitig ebenfalls zurückliegende Glukosewerte verbessert werden.

Weitere Merkmale, Vorteile und weitere Ausführungsformen der Erfindung sind im Folgenden beschrieben oder werden dadurch offenbar.

Gemäß einer vorteilhaften Weiterbildung wird ein Moving Horizon-Schätzverfahren zum Ermitteln des aktuellen Glukosewerts im Schritt d) angewandt auf früher ermittelte Glukosewerte und zumindest einen früheren Gewebe-Glukosewert durchgeführt. Damit wird insbesondere eine effiziente Bestimmung des aktuellen Glukosewerts auf Basis früherer gemessener Glukosewerte ermöglicht.

Gemäß einer weiteren vorteilhaften Weiterbildung wird das Sensormodell in Form einer linearen Funktion zwischen Messwerten und Gewebe-Glukosewerten bereitgestellt. Dies ermöglicht eine besonders effiziente und schnelle Berechnung der interstitiellen Gewebe-Glukosewerte auf Basis der Messwerte der Messreihe bei gleichzeitig ausreichender Genauigkeit.

Gemäß einer weiteren vorteilhaften Weiterbildung wird das Sensormodell in Form einer nichtlinearen Funktion zwischen Messwerten und Gewebe-Glukosewerten bereitgestellt. Hierbei sind beispielsweise die folgenden Sensormodelle bereitstellbar, wobei y den Messwert, IG die Gewebe-Glukosekonzentration und a, b, c beziehungsweise A, b, c Sensorparameter darstellen:

$$y = c - a*b/(IG + b)$$

Alternativ ist auch folgendes nichtlineare Sensormodell möglich:

$$y = (A*b + c*IG)/(IG + b)$$

Vorteil hiervon ist eine höhere Genauigkeit der berechneten interstitiellen Gewebe-Glukosewerte auf Basis der Messwerte der Messreihe, insbesondere bei Sensoreinrichtungen mit Affinitätsbindungssensoren beziehungsweise optischen Sensoren.

Gemäß einer weiteren vorteilhaften Weiterbildung wird der Wert für den Horizont des Moving Horizon-Schätzverfahrens zum Ermitteln des aktuellen Glukosewerts kleiner oder gleich 10 gewählt. Dies ermöglicht eine effiziente Berechnung der interstitiellen Gewebe-Glukosewerte auf Basis der Messwerte der Messreihe bei gleichzeitig hoher Genauigkeit.

Gemäß einer weiteren vorteilhaften Weiterbildung werden eine Varianz des Messrauschens und/oder Prozessrauschens, insbesondere zumindest regelmäßig, geschätzt. Dies ermöglicht eine einfache und schnelle Ermittlung der Rauschwerte und damit insgesamt eine genauere Bestimmung des aktuellen Glukosewerts.

Gemäß einer weiteren vorteilhaften Weiterbildung werden die Varianz des Messrauschens und/oder die Varianz des Prozessrauschens auf Basis zumindest eines früheren jeweiligen Wertes geschätzt, insbesondere interpoliert und/oder gewichtet, vorzugsweise unter Verwendung einer exponentiellen Glättung. Auf diese Weise können die jeweiligen Rauschwerte, die in Abhängigkeit der Zeit variieren, angepasst beziehungsweise aktualisiert werden, was insgesamt die Genauigkeit bei der Bestimmung des aktuellen Glukosewerts weiter verbessert.

Gemäß einer weiteren vorteilhaften Weiterbildung werden nur teilweise benötigte Werte zur Berechnung der Schätzung des Messrauschens und/oder des Prozessrauschens zwischengespeichert und es werden nicht zwischengespeicherte, benötigte Werte anhand der gespeicherten Werte interpoliert. Damit ist es beispielsweise möglich, für die Bestimmung von Messrauschwerten und/oder Prozessrauschwerten notwendige und rechenintensive Werte zumindest teilweise zwischen zu speichern und für spätere Messungen verfügbar zu machen, was insgesamt den Rechenaufwand für die Bestimmung des aktuellen Glukosewerts senkt ohne dessen Genauigkeit wesentlich zu vermindern.

Gemäß einer weiteren vorteilhaften Weiterbildung wird eine Anzahl der früheren jeweiligen Werte, welche größer ist als der Wert für den Horizont des Moving Horizon-Schätzverfahrens, insbesondere zumindest doppelt so groß, vorzugsweise mindestens um den Faktor 5, gewählt. Damit wird die Genauigkeit der Schätzung von Prozessund/oder Messrauschen in Bezug auf den aktuellen Glukosewert festgelegt und verbessert insgesamt die Genauigkeit der Bestimmung oder Quantifizierung des aktuellen Glukosewerts.

Gemäß einer weiteren vorteilhaften Weiterbildung wird die Varianz des Messrauschens und/oder die Varianz des Prozessrauschens regelmäßig angepasst basierend auf dem Wert der Summe von Horizont des Moving Horizon-Schätzverfahrens und der Anzahl der früheren jeweiligen Werte zur Berechnung der Schätzung des Messrauschens und/oder des Prozessrauschens. Damit wird sichergestellt, dass eine effiziente Anpassung der jeweiligen Rauschwerte in regelmäßigen Abständen erfolgt, um einerseits eine ausreichende Genauigkeit des aktuellen Glukosewerts, andererseits um unnötige Anpassungen beziehungsweise Aktualisierungen, die sich nicht oder nur unwesentlich in einer Erhöhung der Genauigkeit des aktuellen Glukosewerts niederschlagen, zu vermeiden.

Gemäß einer weiteren vorteilhaften Weiterbildung werden ermittelte Werte mittels einer Filterfunktion gefiltert, wobei mittels der Filterfunktion Fehler, insbesondere Messfehler, der Sensoreinrichtung unterdrückt werden. Mittels der Filterfunktion können auf einfache Weise fehlerhafte Messungen, beispielsweise Sensorfehler oder Ausreißer bei den Messwerten aussortiert werden, das heißt, sie werden bei der weiteren Berechnung des aktuellen Glukosewerts nicht berücksichtigt.

Gemäß einer weiteren vorteilhaften Weiterbildung werden Messrauschwerte mittels der Filterfunktion gewichtet.

Damit wird ein Unterschätzen und Überschätzen von Messerauschwerten vermieden: ein Unterschätzen führt zu äußerst fehlerbehafteten Signalen beziehungsweise Messwerten, wohingegen ein Überschätzen zu einem zu glatten Verlauf von Messwerten der Messreihen führt. Insgesamt wird damit die Genauigkeit weiter verbessert.

Gemäß einer weiteren vorteilhaften Weiterbildung werden zur Erkennung von Fehlern der Sensoreinrichtung der Gradient des Anstiegs in einem aktuellen GewebeGlukosewert und/oder der aktuelle Gewebe-Glukosewert ausgewertet. Alternativ oder zusätzlich kann dies auch mit aktuellem Glukosewert und/oder dessen Gradienten des Anstiegs in dem aktuellen Glukosewert des Transportfluids durchgeführt werden. Dies ermöglicht eine einfache und gleichzeitig zuverlässige und effiziente Erkennung von Fehlern der Sensoreinrichtung.

Gemäß einer weiteren vorteilhaften Weiterbildung werden ermittelte Messwerte unterhalb einem vorgebbaren unteren und/oder oberhalb einem vorgebbaren oberen Schwellwert mittels der Filterfunktion verworfen, insbesondere wobei der untere und obere Schwellwert zu physiologischen Grenzen korrespondiert, vorzugsweise wobei der untere Schwellwert einen Wert zwischen 10-50 mg/dL, insbesondere 30 mg/dL aufweist und der obere Schwellwert einen Wert zwischen 100-600 mg/dL, vorzugsweise 450 mg/dL, aufweist. Mittels entsprechender Schwellenwerte lassen sich falsche Sensormesswerte, welche hierbei sowohl Glukosemesswerte im Transportfluid, insbesondere im Blut als auch Gewebe-Glukosemesswerte umfassen, in vorteilhafter Weise durch eine Gewichtungsmatrix, höchstvorzugsweise einer diagonalen Gewichtungsmatrix, für die weitere Berechnung ausblenden. Vorzugsweise fungiert die Gewichtungsmatrix derart, dass falsche Messwerte der Sensoreinrichtung mit dem Faktor 0 gewichtet werden, wobei alle anderen Messwerte mit dem Faktor 1 gewichtet werden. Die falschen Messwerte, wie beispielsweise Ausreißer in den Sensormesswerten, werden über die absolute Glukosekonzentration im Transportfluid, insbesondere Blut, sowie deren Änderungsrate beziehungsweise deren Gradienten bestimmt. Im erstgenannten Fall werden vorzugsweise die physiologischen Schranken einer Blutglukosekonzentration eingeführt, wobei ein Blutglukosekonzentrationsbereich von 10 mg/dL bis 600 mg/dL, bevorzugt 20 mg/dL bis 500 mg/dL, besonders bevorzugt 30 mg/dl bis 450 mg/dl, angenommen wird. Messwerte außerhalb dieser physiologischen Schranken werden als falsche Sensormesswerte mit dem Faktor 0 gewichtet. Der Gradient der Glukosekonzentration im Transportfluid, insbesondere im Blut beziehungsweise die Änderungsrate der Glukosekonzentration im Transportfluid, insbesondere im Blut, kann ebenfalls bestimmt und dessen Wert mit einer physiologisch realistischen Änderungsrate verglichen werden. Die betragsmäßige Änderungsrate der Glukosekonzentration im Transportfluid, insbesondere im Blut beträgt demnach einen Wert von 0.1 mg/dL pro min bis 15 mg/dL pro min, bevorzugt einen Wert von 0.5 mg/dL pro min bis 10 mg/dL pro min, höchstbevorzugt einen Wert von 1 mg/dL pro min bis 3 mg/dL pro min.

Gemäß einer weiteren vorteilhaften Weiterbildung erfolgt eine Kalibrierung der Messwerte der Sensoreinrichtung nach dem Durchführen von Schritt d). Damit ist eine Verwendung eines nicht-kalibrierten Gewebe-Glukosemesswerts möglich, was den Vorteil hat, dass die Kalibrierung nicht zwingend vor Durchführen des Moving Horizon-Schätzverfahrens erfolgen muss, sondern ebenfalls nach dem Moving Horizon-Schätzverfahren erfolgen kann. Ein weiterer Vorteil der Verwendung nicht-kalibrierter Gewebe-Glukosemesswerte ist, dass die nicht-kalibrierten Gewebe-Glukosemesswerte eine höhere Korrelation zur Selbst-Überwachungs-Blut-Glukosekonzentration aufweisen und sich dadurch in vorteilhafter Weise beispielsweise die Parameter des Sensormodells einfacher und exakter bestimmen lassen.

Gemäß einer weiteren vorteilhaften Weiterbildung umfasst das Zustandsübergangsmodell ein Diffusionsmodell zur zeitabhängigen Modellierung des Diffusionsprozesses von Glukose von dem Transportfluid ins umgebende Gewebe. Mittels eines Diffusionsmodells, insbesondere basierend auf einer Diffusionskonstante, ist eine einfache und gleichzeitig wenig rechenintensive Modellierung der Dämpfung und zeitlichen Verzögerung zwischen Glukosewert im Transportfluid, insbesondere im Blut und Gewebe-Glukosewert, möglich.

Gemäß einer weiteren vorteilhaften Weiterbildung werden Sensormodellparameter des Sensormodells und/oder Zustandsübergangsparameter des Zustandsübergangsmodells geschätzt und/oder zumindest regelmäßig, aktualisiert. Vorteil hiervon ist, dass damit die Genauigkeit insgesamt zur Bestimmung des aktuellen Glukosewerts erhöht wird, gleichzeitig Parameter des jeweiligen Modells flexibel an sich ändernde Umstände oder Einflüsse angepasst werden können.

Weitere wichtige Merkmale und Vorteile der Erfindung ergeben sich aus den Unteransprüchen, aus den Zeichnungen und aus der dazugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungen und Ausführungsformen der vorliegenden Erfindung sind in den Zeichnungen dargestellt und werden in der nachfolgenden Beschreibung näher erläutert. Sämtliche Umformungsschritte von Gleichungen, Annahmen, Lösungsverfahren, etc. können separat verwendet werden ohne den Rahmen der Erfindung zu verlassen.

Dabei zeigen in schematischer Form

Fig. 1     Schritte eines Verfahrens gemäß einer Ausführungsform der vorliegenden Erfindung;

Fig. 2    Schritte eines Verfahrens gemäß einer Ausführungsform der vorliegenden Erfindung; und

Fig. 3    Vergleich eines Verfahrens gemäß einer Ausführungsform der vorliegenden Erfindung mit bereits bekannten Verfahren.

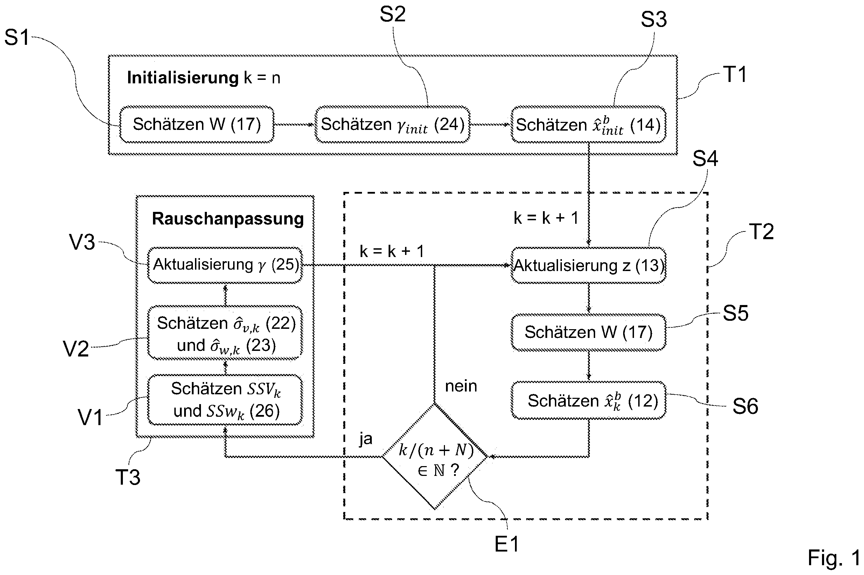

In Figur 1 sind im Detail Schritte zur Ermittlung der Glukosekonzentration im Blut basierend auf dem Moving Horizon-Schätzverfahren gezeigt, wobei die Varianz des Prozessrauschens und des Messrauschens angepasst wird.
In einer Startphase T1 erfolgt die Initialisierung des Verfahrens mittels der nachfolgenden erläuterten Schritte S1-S3. Nach der Initialisierung erfolgt in einer zweite Phase T2 in diskreten Zeitschritten basierend auf dem Moving Horizon-Schätzverfahren, die Ermittlung der Glukosekonzentration im Blut mittels der nachfolgenden erläuterten Schritten S4-S6, sowie einem Entscheidungsschritt E1. Parallel hierzu erfolgt in einer dritten Phase T3 eine Anpassung von Mess- und Prozessrauschen mit den nachfolgend erläuterten Schritten V1-V3.
Bevor im Einzelnen auf die einzelnen Phasen T1-T3 und deren Schritte eingegangen wird, werden zunächst die Grundlagen zur Durchführung des Moving Horizon-Schätzverfahrens im Folgenden erläutert. Das im Folgenden verwendete Moving Horizon-Schätzverfahren ist ein Verfahren zur Zustandsschätzung durch Minimierung einer sogenannten Kostenfunktion, die auf einem gleitenden Zeitausschnitt von n diskreten Zeitschritten ausgeführt wird. Hierbei wird ein diskretes Zeitsystem definiert

$$x_\kappa = f(x_{k-1}, w_{k-1})$$

$$y_\kappa = h(x_k, v_k)$$

wobei $x_\kappa$ der Vektor der Zustandsvariable ist und $y_\kappa$ der Messvektor. Weiterhin umfasst die Kostenfunktion die gewichtete Norm des Messrauschens $v_\kappa$ und Prozessrauschen $w_k$ des Horizonts n zur Zeit k.
Das Optimierungsproblem nimmt damit die folgende Form an:

$$\min_{\{x\}_{j|k}^{k|k}} \sum_{j=k-N+1}^{k} \frac{1}{\sigma_{v,k}} \parallel v_{j|k} \parallel^2 + \frac{1}{\sigma_{w,k}} \parallel w_{j-1|k} \parallel^2$$

$$(2)$$

wobei $\{x\}_{j|k}^{k|k}$ die geschätzten Zustände $x_{k-N+1},...,x_k$ zur Zeit k und $\sigma_{w,k}^2 = \mathrm{var}(w_k)$ und $\sigma_{v,k}^2 = \mathrm{var}(v_k)$ die Varianzen des Prozessrauschens beziehungsweise Messrauschens sind. Prozess- und Messrauschen sind unkorrelliert mit dem Mittelwert 0, jedoch nicht notwendigerweise gaussverteilt.
Zur Modellierung des Diffusionsprozesses zwischen Blut und umliegendem Gewebe wird insbesondere folgender Zusammenhang angenommen:

$$\frac{di(t)}{dt} = \frac{1}{\tau}(b(t) - i(t))$$

$$(3)$$

wobei i(t) das Gewebe-Glukosesignal, b(t) das Blut-Glukosesignal und $\tau$ die Zeitkonstante des Diffusionsprozesses bezeichnet. Das Sensormodell wird insbesondere als lineares Modell angenommen mit Messwert y(t):

$$y(t) = p_0\, i(t) + p_1$$

Es ist jedoch auch möglich, ein nichtlineares Modell zu verwenden, beispielsweise der Form f(ik) = $(p_1 * i_k)/(p_0 + i_k)$.
Unter der Annahme, dass das Ausgabesignal y(t) linear in i(t) ist, kann ein ideales und kalibriertes Blut-Glukosesignal $x^b = p_0\, b(t) + p_1$ und ein ideales und kalibriertes Gewebe-Glukosesignal $x^i = p_0\, i(t) + p_1$ eingeführt werden. Unter

der weiteren Annahme, dass sich die Sensorparameter $p_0$ und $p_1$ nur langsam ändern, gilt das Diffusionsmodell ebenso für die nicht-kalibrierten Signale

$$\frac{dx^i(t)}{dt} = p_0 \frac{di(t)}{dt} = \frac{1}{\tau} (x^b(t) - x^i(t))$$

$$(4)$$

Modelliert man nun diese Gleichung in Zeitschritten $\Delta t$ und die Blut-Glukosekonzentration $x^b$ mit einem autoregressiven Modell, gelangt man zu der folgenden diskreten Zustandsraumdarstellung:

$$x^b_{k+1} = 2x^b_k - x^b_{k-1} + w_k$$

$$x^i_{k+1} = x^i_k + \frac{1}{\tau} (x^b_k - x^i_k)$$

$$y_k = x^i_k + \upsilon_k$$

$$(5)$$

Für das oben genannte alternative nichtlineare Modell würde folgende Zustandsraumdarstellung gelten:

$$b_{k+1} = 2b_k - b_{k-1} + w_k$$

$$i_{k+1} = i_k + \frac{1}{\tau} (b_k - i_k)$$

$$f(i_k) = \frac{p_1 \cdot i_k}{i_k + p_0}$$

$$y_k = f(i_k) + v_k$$

$$w_k = b_{k+1} - 2b_{k-1}$$

$$v_k = y_k - \frac{p_1 i_k}{(p_0 + i_k)}$$

Im Folgenden wird wieder das oben beschriebene lineare Sensormodell angenommen. Sobald also der ideale nicht-kalibrierte Blut-Glukosewert $x^b_k$ geschätzt wird, kann die Blut Glukosekonzentration für das lineare Modell mittels

$$b_k = \frac{y^b_k - p_1}{p_0}$$

$$(6)$$

bestimmt werden.

Das in Gleichung (2) formulierte Moving Horizon-Problem wird nun durch Optimierung des kalibrierten rauschfreien Blut-Glukosesignals

$$x^b_{k-N+1|k}, ..., x^b_{k|k}$$

gelöst. Im Folgenden wird nun eine Matrixschreibweise verwendet mit N-dimensionalen Vektoren, die die vergangenen Zustandsvariablen

$$j = k - N - 1, \ldots, k$$

beziehungsweise Messwerte des Sensors zum Zeitpunkt k umfassen. Das Gewebe-Glukosesignal $x_k^i$ wird daher beschrieben als

$$x_k^i = A x_k^b + B z_k$$

$$(7)$$

wobei $z_k := (x_{k\text{-}N}^i, x_{k\text{-}N\text{-}1}^b, x_{k\text{-}N}^b)^T$ die Anfangszustände und die Matrizen A und B folgendermaßen definiert werden

$$A = \frac{1}{\tau}\begin{pmatrix} 0 & 0 & 0 & \cdots & 0 \\ 1 & 0 & 0 & \cdots & 0 \\ a & 1 & 0 & \cdots & 0 \\ \vdots & \ddots & \ddots & \ddots & \vdots \\ a^{N-2} & \ldots & a & 1 & 0 \end{pmatrix}, B = \begin{pmatrix} a & 0 & a^0 \\ \vdots & \vdots & \vdots \\ a^N & 0 & a^{N-1} \end{pmatrix}$$

mit $\propto = 1 - \frac{1}{\tau}$. Das Messerauschen $\upsilon_k = (\upsilon_{k\text{-}N+1|k}, \ldots, \upsilon_{k|k})^T$ ist gegeben durch

$$\upsilon_k = y_k - x_k^i$$

$$(8)$$

und das Prozessrauschen $w_k = (w_{k\text{-}N|k}, \ldots, W_{k\text{-}1|k})^T$ wird definiert als

$$w_k = \underbrace{\begin{pmatrix} 0 & 0 & 0 & \cdots & 0 \\ -2 & 1 & 0 & \cdots & 0 \\ 1 & -2 & 1 & \cdots & 0 \\ \vdots & \ddots & \ddots & \ddots & \vdots \\ 0 & \ldots & 1 & -2 & 1 \end{pmatrix}}_{C \in R^{N \times N}} x_k^b + \underbrace{\begin{pmatrix} 0 & 1 & -2 \\ 0 & 0 & 1 \\ \vdots & \ddots & \vdots \\ 0 & \ldots & 0 \end{pmatrix}}_{D \in R^{N \times 3}} z_k$$

$$(9)$$

Die Formulierung des Moving Horizon-Problems für das lineare Sensormodell ergibt sich dann durch

$$\min_{x_k^b}(\parallel w_k \parallel_{Q_k^{-1}}^2 + \parallel v_k \parallel_{R_k^{-1}}^2)$$

$$(10)$$

mit dem Gewichtsmatrizen $Q_k = \text{cov}(w_k)$ und $R_k = \text{cov}(\upsilon_k)$, die den Kovarianzmatrizen des Prozessrauschen und des Messrauschen entsprechen. Für das alternative, beschriebene nichtlineare Sensormodell ergibt sich das folgende Optimierungsproblem:

$$\min_{b_{k-N}\ldots b_k} \left\{ \sum_{j=k-N-1}^{k-1} \| \ w_k \ \|^2_{\frac{1}{\sigma_w^2}} + \sum_{j=k-N}^{k} \| \ v_n \ \|^2_{\frac{1}{\sigma_v^2}} \right\},$$

wobei die Varianzen $\sigma_w^2$ und $\sigma_v^2$ ermittelt beziehungsweise festgelegt werden müssen. Dieses Optimierungsproblem kann im Allgemeinen nicht direkt gelöst werden, sondern mittels iterativer Lösungsverfahren gelöst werden. Die Bestimmung beziehungsweise Aktualisierung der Sensorparameter des nichtlinearen Modells ergibt sich dann aus den Selbst-Überwachungs-Messungen $bg_i(t=t_i)$:

$$\min_{p_0,p_1} \sum_{i=1}^{N} \| \ bg_i - \hat{b} \ (t = t_i, p_0, p_1) \ \|^2$$

Im Folgenden wird nun wieder das lineare Sensormodell angenommen. Nach Einsetzen der Gleichungen (8) und (9) in das Moving Horizon-Problem für das lineare Modell, ergibt sich das folgende quadratische Problem:

$$\min_{x_k^b} \left\{ x_k^{b^T} (A^T R^{-1} A + C^T Q^{-1} C) x_k^b - \left( (y_k - Bz_k)^T R^{-1} A - z_k^T D^T Q^{-1} C \right) x_k^b \right\}$$

$$(11)$$

was durch Matrixinversion

$$\hat{x}_k^b = (A^T R^{-1} A + C^T Q^{-1} C)^{-1} \cdot (A^T R^{-1}(y_k - Bz_k) - C^T Q^{-1} Dz_k) = Hy_k + Gz_k$$

$$(12)$$

gelöst werden kann oder unter Berücksichtigung von bekannten Verfahren zur Lösung quadratischer Optimierungsprobleme.

Die Anfangszustände $z_k$ werden entsprechend der Lösung der vorherigen Schätzschritte festgelegt:

$$z_k = \begin{pmatrix} x_{k-N}^i \\ x_{k-N-1}^b \\ x_{k-N}^b \end{pmatrix} = \begin{pmatrix} \hat{x}_{k-N|k-1}^i \\ \hat{x}_{k-N-1|k-2}^b \\ \hat{x}_{k-N|k-1}^b \end{pmatrix} \cdot$$

$$(13)$$

Im Falle der Initialisierung mit k = N müssen die Anfangszustände geschätzt werden. Dementsprechend wird der Optimierungsvektor $x_{init} = (z_k, x_k^b)$ um die Anfangspunkte erweitert. Das Optimierungsproblem kann dann entsprechend mit $A_{init}$ = [BA] und $C_{init}$ = [DC] und durch Ersetzen von $B_{init}$ und $D_{init}$ mit Nullmatrizen umgeschrieben werden.

Die Lösung des geänderten Optimierungsproblems lautet dann

$$\hat{x}_{init} = (A_{init}^T R_{init}^{-1} A_{init} + C_{init}^T Q_{init}^{-1} C_{init})^{-1} \underbrace{A_{init}^T y_k}_{H_{init}}$$

$$\text{(14)}$$

Im Gegensatz zu anderen Schätzverfahren wird mittels des Moving Horizon-Schätzverfahrens sowohl ein aktueller Wert $\hat{x}_{k|k}^b$ als auch frühere Werte $\hat{x}_{j|k}^b$ $(j > k\text{-}N\text{+}1)$ geschätzt. Das Aktualisieren des Blut-Glukosewerts beziehungsweise -signals, nicht nur mit dem aktuellen Schätzwert, sondern ebenfalls mit den Schätzwerten im gesamten (zurückliegenden) Horizont/Zeitfenster $\hat{x}_{j|k}^b$ $(j > k\text{-}N\text{+}1)$ führt zu

$$\hat{X}^b(k\text{-}N\text{+}1, ..., k) = \hat{x}_k^b$$

$$\hat{X}^i(k\text{-}N\text{+}1, ..., k) = \hat{x}_k^i = A\hat{x}_k^b + Bz_k.$$

$$\text{(15)}$$

Hierbei umfasst $\hat{X}^b(i)$ für $i \le k\text{-}N$ nur die Schätzwerte

$$\hat{x}_{k\text{-}N\text{+}1|k}^b$$

Da CGM-Systeme typischerweise empfindlich gegenüber mechanischen Störungen sind, die zu fehlerhaften Messwerten führen können, können entsprechende fehlerhafte Sensor-Messwerte durch Gewichten des Messrauschens mit einer Gewichtsmatrix W berücksichtigt werden.

In den oben beschriebenen Gleichungen (11) und (12) kann eine gewichtete, inverse Kovarianzmatrix

$$R^{-1} = \frac{1}{N} \sum_{i=k-N}^{k} w_i \underbrace{\begin{pmatrix} w_k & & 0 \\ & \ddots & \\ 0 & & w_{k-N} \end{pmatrix}}_{W} R^{-1}$$

$$\text{(16)}$$

eingeführt werden, um die Lösung des Optimierungsproblems schätzen zu können. Hierbei werden die diagonalen Einträge der Gewichtsmatrix W entsprechend fehlerhaften Messwerten auf 0 gesetzt:

$$w_i = \begin{cases} 0 & \text{sensor error or outliers} \\ 1 & \text{else} \end{cases}$$

$$\text{(17)}$$

Um Sensorfehler oder Messausreißer zu detektieren, kann insbesondere der Gradient im entsprechenden Gewebe-Glukosewert sowie der aktuelle Gewebe-Glukosewert selbst herangezogen werden. Alternativ oder zusätzlich ist es möglich, obere beziehungsweise untere Schwellwerte der Messsignale beziehungsweise Messwerte des Sensors zu verwenden und Messwerte, die außerhalb von unterem und oberem Schwellwerte liegen, als fehlerhaft zu klassifizieren.

Zur effizienteren Durchführung des Moving Horizon-Schätzverfahrens ist es insbesondere erforderlich, die

Varianz des Messrauschens $\sigma^2_{\upsilon,k}$ und des Prozessrauschens $\sigma^2_{w,k}$ zu kennen. Im Allgemeinen sind diese zwei Parameter unbekannt und müssen geschätzt werden. Darüber hinaus ändern sich diese Parameter im Laufe der Zeit. Eine Anpassung beziehungsweise eine Aktualisierung der Varianzen führt daher direkt zu einer Änderung, insbesondere einer Verbesserung der Qualität der Schätzung durch das Moving Horizon-Schätzverfahren. Wird allerdings das Messrauschen zu gering eingeschätzt, führt dies zu einem sehr rauschbehafteten Messsignal und damit zu fehlerhaften Messwerten. Wird andererseits das Messrauschen zu hoch eingeschätzt beziehungsweise das Prozessrauschen zu gering eingeschätzt, führt dies zu einer zeitverzögerten Schätzung, was ebenfalls die Genauigkeit der Bestimmung des aktuellen Glukosewerts reduziert.

Im Folgenden wird nun ein Verfahren beschrieben, um die Messrauschvarianz des Messrauschens und die Prozessrauschvarianz des Prozessrauschens vorherzusagen. Darüber hinaus wird im Folgenden ein Verfahren zur einfachen Anpassung beziehungsweise Aktualisierung der jeweiligen Varianzen beschrieben.

Grundlage für dieses Verfahren ist, dass jeder Freiheitsgrad äquivalent jedem anderen Freiheitsgrad ist, wie dies beispielsweise in der Nicht-Patentliteratur von Grace Wahba "Bayesian "Confidence Intervals" for the Cross-Validated Smoothing Spline" (Journal oft he Royal Statistical Society: Series B (Methodological) 45, (1983), 133-150) beschrieben ist.

Unter der Annahme, dass das Prozessrauschen $w_{j-1|k}$ und das Messrauschen $\upsilon_{j|k}$ eines Horizonts der Länge n und j=k-n + 1, ...k Teil einer Verteilung mit Varianz $\sigma_{w,k}$ beziehungsweise $\sigma_{\upsilon,k}$ sind, entsprechen die Kovarianzmatrizen $R_k$ und $Q_k$

$$R_k = \sigma^2_{v,k}I \qquad \text{and} \qquad Q_k = \sigma^2_{w,k}I.$$

Gleichung (12) vereinfacht sich damit zu

$$H(\gamma_k) = (A^TA+ \gamma_k C^TC)^{-1}A^T$$

und

$$G(\gamma_k) = (A^TA+ \gamma_k C^TC)^{-1}(B-C^TD)$$

wobei $\gamma_k = \frac{\sigma^2_{v,k}}{\sigma^2_{w,k}}$ der Quotient der Varianzen des Prozessrauschens und des Messrauschens ist.

Im nächsten Schritt werden die Gleichungen (7) und (12) in die Definition des Messrauschens (8) und des Prozessrauschens (9) eingesetzt:

$$w=CH(\gamma_k)y_k+(CG+D)z_k$$

$$\upsilon=\big(I-AH(\gamma_k)\big)y_k-(AG+B)z_k$$

Unter der Annahme, dass die Varianz der Startpunkte $z_k$ gleich Null ist, haben die Kovarianzmatrizen die folgende Form

$$\mathrm{cov}(w_k) = CH(\gamma_k)\, \mathrm{cov}(y_k)\, H^T(\gamma_k)C^T$$

$$\mathrm{cov}(\upsilon_k) = (I-AH(\gamma_k))\, \mathrm{cov}(y_k)\, (I-AH(\gamma_k))^T$$

$$(18)$$

Darüber hinaus hängt die Kovarianz des nicht kalibrierten, rauschfreien Blut-Glukosesignals $\mathrm{cov}(x_k^b) = \sigma^2_w C^{-1}(C^{-1})^T$ und die Kovarianz des idealen, nicht-kalibrierten Gewebe-Glukosesignals

$$\text{cov}(x_k^b) = \sigma_w^2 AC^{-1}(C^{-1})^T A^T$$

nur von der Varianzmatrix des Prozessrauschens ab. Da die Kovarianzmatrix des Messsignals sich aus der Kovarianzmatrix des Messrauschens und der des nicht-kalibrierten, rauschfreien Gewebe-Glukosesignals zusammensetzt folgt damit

$$\text{cov}(y_k) = \sigma_v^2 I + \sigma_w^2 A(C^{-1})^T C^{-1} A \qquad (19)$$

Setzt man nun Gleichung (19) in Gleichung (18) ein und führt eine Matrixinversion aus, ergibt sich die Kovarianzmatrix des Prozessrauschens und des Messrauschens folgendermaßen:

$$\text{cov}(v_k) = \sigma_v^2 (I\text{-}AH(\gamma_k)) \qquad (20)$$

$$\text{cov}(w_k) = \sigma_w^2 CH(\gamma_k)AC^{-1} \qquad (21)$$

Der erwartete Wert der Summe der quadrierten Messfehler $SSV_k = v_k^T v_k$ kann umgeschrieben werden zu

$$E(SSV_k) = E(v_k^T v_k) = E\left(\text{tr}(v_k v_k^T)\right) = E(\text{tr}(\text{cov}(v_k)))$$

Die Varianz des Messrauschens ergibt sich dann folgendermaßen:

$$\sigma_{v,k}^2 = \frac{E(SSV_k)}{N - s(\gamma_k)}$$

wobei $s(\gamma_k) = \text{tr}(AH(\gamma_k))$ ist.

Damit wird ein konsistenter Schätzwert für die Varianz des Messrauschens $\widehat{\sigma}_{v,k}^2$ ermöglicht:

$$\widehat{\sigma}_{v,k}^2 = \frac{SSV_k}{N\text{-}s(\gamma_k)} \qquad (22)$$

Die Varianz des Prozessrauschens kann in ähnlicher Weise wie die Varianz des Messrauschens ermittelt werden:

$$\widehat{\sigma}_{w,k}^2 = \frac{SSW_k}{s(\gamma_k)} \qquad (23)$$

Insoweit werden die erwarteten Werte der Summe der quadratischen Prozessfehler $SSW_k = w_k^T w_k$ und Gleichung (21) benutzt. Da der Wert $\gamma_k$ die Gleichung

$$\hat{\gamma}_k = \frac{\hat{\sigma}_{v,k}^2}{\hat{\sigma}_{w,k}^2} = \frac{s(\hat{\gamma}_k)SSV_k(\hat{\gamma}_k)}{\left(n - s(\gamma_k)\right)SSW_k(\hat{\gamma}_k)}$$

erfüllen muss, wird das optimale $\hat{\gamma}_{init}$ mit einem ableitungsfreien Optimierungsverfahren geschätzt, wie beispielsweise in der Nicht-Patentliteratur Rios und Sahinidis "Derivative-free optimization: a review of algorithms and comparison of software implementations" (Journal of Global Optimization 56,3 (2013), 1247-1293) beschrieben:

$$\min_{\gamma} \left\| \gamma - \frac{s(\gamma)\,SSV\,(\gamma, x_{init}^b)}{\left(n - s(\gamma)\right)SSW\,(\gamma, x_{init}^b)} \right\|^2 \tag{24}$$

Danach wird $\hat{\gamma}_k = \frac{\tilde{\sigma}_{v,k}^2}{\tilde{\sigma}_{w,k}^2}$ angepasst und das Prozessrauschen und das Messrauschen nach n + N Zeitschritten

$$\tilde{\sigma}_{v,k}^2 = (1-\eta)\hat{\sigma}_{v,k}^2 + \eta\,\tilde{\sigma}_{v,k-n}^2$$

$$\tilde{\sigma}_{w,k}^2 = (1-\eta)\hat{\sigma}_{w,k}^2 + \eta\,\tilde{\sigma}_{w,k-n}^2 \tag{25}$$

unter Benutzung eines Glättungsfaktors $\eta$ aktualisiert.

Die Länge des Rausch-Anpassungshorizonts n muss nicht mit dem Schätzhorizont N übereinstimmen. Ein langer Schätzhorizont N erhöht den Rechenaufwand erheblich, zeigt jedoch lediglich eine leicht verbesserte Schätzgenauigkeit. Da die Schätzgenauigkeit der Varianzen stark mit der Anzahl der Datenpunkte korreliert, wird insbesondere der Rauschanpassungshorizont n wesentlich größer als der Schätzhorizont N gewählt.

Die Varianzen $\hat{\sigma}_{w,k}^2$ und $\hat{\sigma}_{v,k}^2$ werden anhand der Gleichungen (22), (23) und der Summe der quadrierten Prozessfehler beziehungsweise entsprechende Summe der quadrierten Messfehler geschätzt:

$$SSV_k = \sum_{j=k-n+1}^{k} \hat{X}^b(j) - 2\hat{X}^b(j-1) + \hat{X}^b(j-2)$$

$$SSW_k = \sum_{j=k-n+1}^{k} \hat{X}^i(j) - y(j). \tag{26}$$

Im Fall von falschen Sensormesswerten werden die Rauschvarianzen insbesondere nicht aktualisiert, da dies zu einer fehlerhaften Schätzung der Prozess- und Messvarianzen $\hat{\sigma}_{v,k}^2$ und $\hat{\sigma}_{w,k}^2$ führen kann.

Um $s(\gamma_k)=tr(AH(\gamma_k))$ schätzen zu können, ist ein hoher Rechenaufwand erforderlich. Da die Matrix A nur von den vordefinierten Matrizen A und C und von $\gamma$ abhängt, ist es möglich, eine Tabelle für $\gamma$ anzulegen in dem relevanten Bereich des Quotienten und für die Berechnung von s für den aktuellen Wert von $\gamma$ eine Interpolation der Werte der Tabelle zu benutzen. Damit kann auch auf einem Computer, Rechner oder auf einem Tablett mit geringerer Rechenleistung eine zufriedenstellende und hinreichend genaue Schätzung in kurzer Zeit ermöglicht werden.

Zusammenfassend wird somit insbesondere in einem ersten Schritt S1 während der Initialisierungsphase T1 die Matrix W gemäß Gleichung (17) geschätzt. Auf Basis der Matrix W wird dann in einem zweiten Schritt S2 das Verhältnis der Varianzen von Prozessrauschen und Messrauschen gemäß Gleichung (24) geschätzt. In einem dritten Schritt S3 wird dann der Startwert gemäß Gleichung (14) geschätzt.

Auf Basis des geschätzten Startwertes werden dann in einem vierten Schritt S4 während der Schätzphase T2 zunächst gemäß Gleichung (13) die Startzustände bestimmt und anhand der Startzustände wiederum analog zum Schritt S1 in einem fünften Schritt S5 gemäß Gleichung (17) die Matrix W geschätzt. In einem sechsten Schritt S6 wird dann die Lösung des Minimierungsproblems gemäß Gleichung (12) berechnet. Anschließend wird im Schritt E1 ermittelt, ob der Quotient aus der Zeit k und der Summe aus dem Schätzhorizont N und dem Rausch-Anpassungshorizont n eine ganze Zahl größer gleich eins ergibt oder nicht. Ist dies nicht der Fall wird der Zeitindex k um eins erhöht und dann erneut die Schritte S4 bis S6 sowie E1 durchgeführt. Ist dies umgekehrt der Fall, wird eine Rauschanpassung T3 mit den Schritten V1 bis V3 durchgeführt. Bei der Rauschanpassung T3 werden in einem ersten Schritt V1 gemäß Gleichung (26) die Summen der quadrierten Prozessfehler beziehungsweise Messfehler ermittelt. Anhand dieser werden dann die entsprechenden Varianzen von Messrauschen und Prozessrauschen gemäß den Gleichungen (22) und (23) in einem zweiten Schritt V2 ermittelt und anschließend der Wert für $\gamma$ gemäß Gleichung (25) aktualisiert. Danach werden wiederum die Schritte S4 bis S6 sowie E1 durchgeführt.

Auf Basis des geschätzten Startwertes werden dann in einem vierten Schritt S4 während der Schätzphase T2 zunächst gemäß Gleichung (13) die Startzustände bestimmt und anhand der Startzustände wiederum analog zum Schritt S1 in einem fünften Schritt S5 gemäß Gleichung (17) die Matrix W geschätzt. In einem sechsten Schritt S6 wird dann die Lösung des Minimierungsproblems gemäß Gleichung (12) berechnet. Anschließend wird im Schritt E1 ermittelt, ob der Quotient aus der Zeit k und der Summe aus dem Schätzhorizont N und dem Rausch-Anpassungshorizont n eine ganze Zahl größer gleich eins ergibt oder nicht. Ist dies nicht der Fall wird der Zeitindex k um eins erhöht und dann erneut die Schritte S4 bis S6 sowie E1 durchgeführt. Ist dies umgekehrt der Fall, wird eine Rauschanpassung T3 mit den Schritten V1-V3 durchgeführt. Hierbei werden in einem ersten Schritt V1 gemäß Gleichung (26) die Summen der quadrierten Prozessfehler beziehungsweise Messfehler ermittelt. Anhand dieser werden dann die entsprechenden Varianzen von Messrauschen und Prozessrauschen gemäß den Gleichungen (22) und (23) in einem zweiten Schritt V2 ermittelt. Anschließend wird der Wert für $\gamma$ gemäß Gleichung (25) aktualisiert. Danach werden wiederum die Schritte S4 bis S6 sowie E1 durchgeführt.

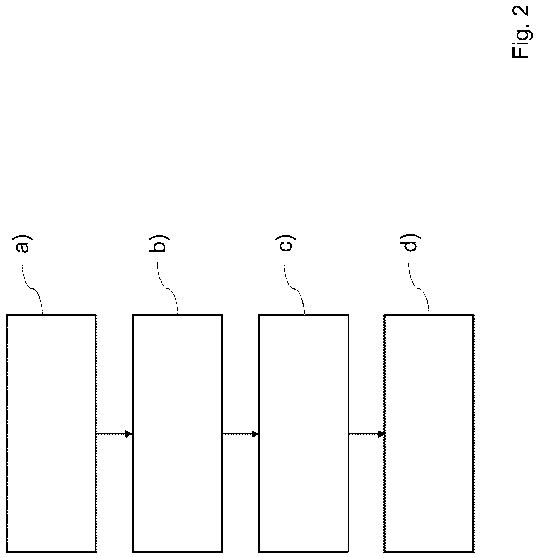

In Figur 2 sind Schritte eines Verfahrens gemäß einer Ausführungsform der vorliegenden Erfindung gezeigt.

Im Detail ist in Figur 2 ein Verfahren zum insbesondere kontinuierlichen Bestimmen eines aktuellen Glukosewerts in einem Transportfluid, insbesondere Blut, eines Organismus, gezeigt. Das Verfahren umfasst dabei die folgenden Schritte:

In einem Schritt a) erfolgt ein Ermitteln einer Messreihe, umfassend zumindest zwei zeitlich beabstandete Messwerte, mittels einer Sensoreinrichtung, für einen Gewebe-Glukosewert in dem das Transportfluid umgebende Gewebe.

In einem weiteren Schritt b) erfolgt ein Ermitteln des Gewebe-Glukosewerts anhand der ermittelten Messreihe basierend auf einem Sensormodell, wobei mittels eines Sensormodells Messwerte der Sensoreinrichtung Gewebe-Glukosewerten unter Berücksichtigung von Messrauschen zugeordnet werden.

In einem weiteren Schritt c) erfolgt ein Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen.

In einem weiteren Schritt d) erfolgt ein Ermitteln des aktuellen Glukosewerts basierend auf dem bereitgestellten Zustandsübergangsmodell und dem ermittelten Gewebe-Glukosewert, wobei zumindest Schritt d), insbesondere die Schritte b)-d) unter Verwendung zumindest eines Moving Horizon-Schätzverfahrens durchgeführt wird.

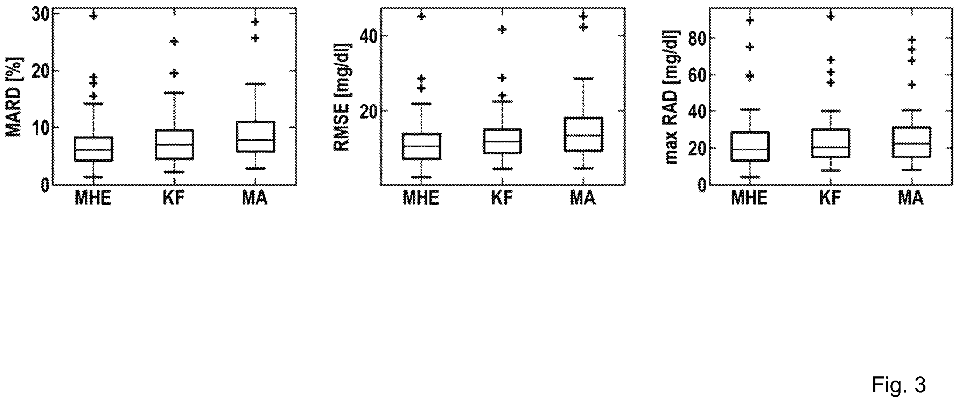

Figur 3 zeigt einen Vergleich eines Verfahrens gemäß einer Ausführungsform der vorliegenden Erfindung mit bereits bekannten Verfahren.

Im Folgenden wird nun ein Vergleich verschiedener Blut-Glukoseschätzverfahren erläutert und das Ergebnis gemäß der Figur 3 dargestellt. Im Einzelnen werden die geschätzten Verfahren mit derselben Sensoreinrichtung - Fiber sense - bekannt aus der Nicht-Patentliteratur Küster, Nikolaus et al. "First Clinical Evaluation of a New Percutaneous Optical Fiber Glukose Sensor for Continuous Glucose Monitoring in Diabetes" (Journal of Diabetes Science and Technology 7, 1 (2014), 13-23), die auf der Basis von Fluoreszenz-Messungen den Blut-Glukosegehalt ermittelt und eine Abtastrate alle 2 Minuten aufweist, durchgeführt. Eine Schätzung der Blut-Glukosekonzentration mittels des Moving Horizon-Schätzverfahrens gemäß einer Ausführungsform der vorliegenden Erfindung wird dabei verglichen mit zwei anderen Schätzverfahren, der Kalman-Filterung KF sowie mit einem geglätteten Sensorsignal mit einem gleitenden Durchschnittsfilter MA. Zusätzlich wird der Effekt von verschiedenen Parametern auf die Blut-Glukoseschätzung erläutert. Die Daten wurden dabei anhand von acht Typ 1 und acht Typ 2 Diabetes-Patienten gesammelt, wobei der entsprechende CGM-Sensor der Sensoreinrichtung über 28 Tage getragen wurde. An den Tagen 1, 7, 15 und 28 wurden Referenzdaten alle 10 Minuten über eine Zeit von 4 Stunden unter Benutzung von Yellow Springs Instrument (YSI) 2300 STAT Plus Glukose analyzer (YSI Life Sciences, Yellow Springs, OH) ermittelt.

Weiterhin wurde der Horizont für das Moving Horizon-Schätzverfahren auf N = 10 und der Horizont für die Rauschanpassung auf n = 50 gesetzt. Die Kalman-Filterung basiert dabei auf der Gleichung (5). Eine Diffusionskonstante respektive Zeitkonstante von $\tau$ = 6 Minuten wurde für beide Verfahren angenommen. Insgesamt wurden die gefilterten Signale des CGM-Systems, also $\hat{x}_k^b$ , erzeugt mittels des Moving Horizon-Schätzverfahrens, ebenso wie mittels des Kalman-Filters und dem mittels des gleitenden Durchschnitt geglätteten Signal verglichen, basierend auf ihrer jeweiligen Übereinstimmung mit den gemessenen Referenzdaten während der klinischen Überwachung. Zur Auswertung der drei unterschiedlichen Schätzverfahren werden drei Auswerteparameter benutzt, zum einen die mittlere absolute relative Differenz (MARD), die Wurzel des mittleren quadratischen Fehlers (RMSE) und die maximale relative absolute Differenz (maxRAD) der vier klinischen Messungen aller 16 Patienten.

In der folgenden Tabelle 1 werden zur Auswertung der Median, das 25 % Quartil - Q1 - und das 75 % Quartil - Q3 - der entsprechenden drei Auswerteparameter aufgeführt.

| method | MARD [%] | RMSE [mg/dl] | maxRAD [mg/dl] |
| --- | --- | --- | --- |
| MHE | 6.1 [4.3, 9.3] | 8.2 [5.7, 11.2] | 19.0 [13.0, 29.5] |
| KF | 7.1 [4.6, 9.6] | 9.6 [6.9, 12.5] | 20.0 [14.9, 31.2] |
| MA | 7.8 [5.3, 11.6] | 11.6 [8.1, 15.2] | 22.8 [14.3, 31.9] |

Zu erkennen ist, dass das Moving Horizon-Schätzverfahren zu den besten Resultaten aller drei Auswerteparameter führt, gefolgt von dem Kalman-Filtersignal KF. Das mittels des gleitenden Durchschnitts geglättete Signal MA, welches ein gefiltertes Gewebe-Glukosesignal repräsentiert, berücksichtigt nicht den Diffusionsprozess zwischen der Blut-Glukosekonzentration und der Gewebe-Glukosekonzentration und führt zu entsprechend schlechten Ergebnissen.

Zur Darstellung des Einflusses der Kalibrierung des Sensors werden nachfolgend unterschiedliche Kalibrierungsverfahren erläutert und miteinander verglichen.

Hierbei können zurückliegende Schätzergebnisse $\hat{x}^b_{k-N+1|k}$ verwendet werden. Dieses Signal wird im Folgenden als $\hat{X}^b$-Signal pMHE, welches frühere Schätzergebnisse enthält, bezeichnet und liefert kleinere Parameter verglichen mit den Ergebnissen des Moving Horizon-Signals, welches nur die aktuellen Schätzwerte $\hat{x}^b_{k|k}$ (median MARD = 5,1%, median RMSE = 7,1 mg/dL und median maxRAD = 14,2 mg/dL) umfasst.

Die früheren, geschätzten Werte $\hat{x}^b_{k-N+1|k}$ verbessern demgemäß die Blut-Glukosemessungen und führen zu einer Verbesserung der Sensorkalibrierung. Die erhöhte Genauigkeit resultiert aus einer verbesserten Berücksichtigung der Zeitverzögerung. Ein "Darüber hinaus Schießen" aufgrund von schnellen Änderungen in der Blut-Glukosekonzentration oder aufgrund von Rauschen wird ebenfalls reduziert. Der Einfluss auf die Genauigkeit der Blut-Glukoseschätzung anhand der CGM-Signale auf den Kalibrierfehler wird im Folgenden beschrieben. Hierbei wird ein sogenanntes Zweipunkt-Kalibrierungs-Verfahren angewendet. Zwei Referenz-Messwerte $b_1$, $b_2$) und die zeitlich korrespondierenden Blut-Glukoseergebnisse $(\hat{x}^b_1, \hat{x}^b_2)$ werden benutzt, um die Sensorparameter gemäß

$$p_0 = \frac{b_2 - b_1}{\hat{x}^b_2 - \hat{x}^b_1} \quad \text{and} \quad p_1 = b_1 - p_0 \hat{x}^b_1$$

zu berechnen. Jede Referenz-Kombination und für jedes geschätzte, nicht-kalibrierte Blut-Glukosesignal (MHE, pMHE, KF und MA) werden die Sensorparameter identifiziert und die Blut-Glukosekonzentration berechnet. Die folgende Tabelle 2

| method | MARD [%] | RMSE [mg/dl] |
| --- | --- | --- |
| pMHE | 10.1 [5.5, 21.5] | 18.2 [10.5, 37.2] |
| MHE | 12.0 [6.8, 25.6] | 21.5 [12.7, 43.2] |
| KF | 12.6 [7.4, 26.7] | 23.0 [14.1, 45.0] |
| MA | 14.3 [8.3, 29.3] | 26.0 [16.5, 50.0] |

zeigt Mediane und Quartile der Verfahren MARD und RMSE für alle möglichen Kalibrierungen. Aus der Tabelle 2 kann entnommen werden, dass das Verfahren pMHE den kleinsten Median und die kleinste interquartile Entfernung von MARD und RMSE aufweist.

Zusammenfassend stellt zumindest eine der Ausführungsformen der Erfindung zumindest einen der folgenden Vorteile und/oder Merkmale bereit:

- Kompensation der Zeitverzögerung durch Modellierung des Diffusionsprozesses und Schätzung des Blutzuckers auf bewegtem, zurückliegenden Horizont (Moving Horizon-Schätzverfahren)
- Beschränkung auf physiologischen Bereich garantiert Robustheit gegenüber Ausreißern im Messsignal
- Adaptive Bestimmung des Regularisierungsfaktors des Problems verbindet adaptive Schätzung des Messrauschens und des Zustandsrauschens
- Adaption sich langsam verändernder Modellparamater zusätzlich möglich
- Effiziente Implementierung gewährleistet hohe Genauigkeit bei geringem Rechenaufwand
- Zeit- und recheneffiziente Schätzung des Blutzuckers auf zurückliegendem Horizont
- Adaption von Modellparametern
- Erhöhung der Robustheit der Schätzung durch die Einführung von Beschränkungen
- Flexibilität hinsichtlich des Sensormodells, beispielsweise können auch nichtlineare Sensormodelle verwendet werden
- Geringer Rechenaufwand zur Schonung der begrenzten Akkulaufzeit
- Beschränkung auf physiologischen Bereich garantiert physiologisch sinnvolle Lösung
- Optimierung des zurückliegenden Horizonts verbessert die Schätzung des Blutzuckers für die Kalibrierung

**Patentansprüche**

1. Computerimplementiertes Verfahren zum insbesondere kontinuierlichen Bestimmen eines aktuellen Glukosewerts in einem Transportfluid, insbesondere Blut, eines Organismus, umfassend die Schritte

    a) Ermitteln, mittels einer Sensoreinrichtung, einer Messreihe, umfassend zumindest zwei zeitlich beabstandete Messwerte, für einen Gewebe-Glukosewert in dem das Transportfluid umgebende Gewebe,
    b) Ermitteln (T2) des Gewebe-Glukosewerts anhand der ermittelten Messreihe basierend auf einem Sensormodell, wobei mittels eines Sensormodells Messwerte der Sensoreinrichtung Gewebe-Glukosewerten unter Berücksichtigung von Messrauschen (V1, V2, V3, T3) zugeordnet werden,
    c) Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen (V1, V2, V3, T3), und
    d) Ermitteln (S6) des aktuellen Glukosewerts basierend auf dem bereitgestellten Zustandsübergangsmodell und dem ermittelten Gewebe-Glukosewert, **dadurch gekennzeichnet, dass** zumindest Schritt d), insbesondere die Schritte b)-d) unter Verwendung zumindest eines Moving Horizon-Schätzverfahrens durchgeführt wird, vorzugsweise wobei ein Moving Horizon-Schätzverfahren zum Ermitteln des aktuellen Glukosewerts im Schritt d) angewandt auf früher ermittelte Glukosewerte und zumindest einen früheren Gewebe-Glukosewert durchgeführt wird.

2. Verfahren gemäß Anspruch 1, **dadurch gekennzeichnet, dass** das Sensormodell in Form einer linearen Funktion oder einer nichtlinearen Funktion zwischen Messwerten und Gewebe-Glukosewerten bereitgestellt wird.

3. Verfahren gemäß einem der Ansprüche 1-2, **dadurch gekennzeichnet, dass** der Wert für den Horizont des Moving Horizon-Schätzverfahrens zum Ermitteln des aktuellen Glukosewerts kleiner oder gleich 10 gewählt wird.

4. Verfahren gemäß einem der Ansprüche 1-3, **dadurch gekennzeichnet, dass** eine Varianz des Messrauschens und/oder eine Varianz des Prozessrauschens, insbesondere zumindest regelmäßig, geschätzt werden, vorzugsweise wobei die Varianz des Messrauschens und/oder die Varianz des Prozessrauschens auf Basis zumindest eines früheren jeweiligen Wertes geschätzt wird, insbesondere interpoliert und/oder gewichtet wird, vorzugsweise unter Verwendung einer exponentiellen Glättung.

5. Verfahren gemäß Anspruch 4, **dadurch gekennzeichnet, dass** nur teilweise benötigte Werte zur Berechnung der Schätzung des Messrauschens und/oder des Prozessrauschens zwischengespeichert werden und nichtzwischengespeicherte benötigte Werte anhand der gespeicherten Werte interpoliert werden.

6. Verfahren gemäß Anspruch 3 und 4, **dadurch gekennzeichnet, dass** eine Anzahl der früheren jeweiligen Werte größer gewählt wird als der Wert für den Horizont des Moving Horizon-Schätzverfahrens, insbesondere zumindest doppelt so groß, vorzugsweise mindestens um den Faktor 5.

7. Verfahren gemäß Anspruch 4 und 6, **dadurch gekennzeichnet, dass** die Varianz des Messrauschens und/oder die Varianz des Prozessrauschens regelmäßig angepasst wird basierend auf dem Wert der Summe vom Horizont des Moving Horizon-Schätzverfahrens und der Anzahl der früheren jeweiligen Werte zur Berechnung der Schätzung des Messrauschens und/oder des Prozessrauschens.

8. Verfahren gemäß einem der Ansprüche 1-7, **dadurch gekennzeichnet, dass** ermittelte Werte mittels einer Filterfunktion gefiltert werden, wobei mittels der Filterfunktion Fehler, insbesondere Messfehler, der Sensoreinrichtung unterdrückt werden, vorzugsweise wobei Messrauschwerte mittels der Filterfunktion gewichtet werden.

9. Verfahren gemäß Anspruch 8, **dadurch gekennzeichnet, dass** zur Erkennung von Fehlern der Sensoreinrichtung der Gradient des Anstiegs in einem aktuellen Gewebe-Glukosewert und/oder der aktuelle Gewebe-Glukosewert ausgewertet werden.

10. Verfahren gemäß einem der Ansprüche 8 oder 9, **dadurch gekennzeichnet, dass** ermittelte Messwerte unterhalb einem vorgebbaren unteren und/oder oberhalb einem vorgebbaren oberen Schwellwert mittels der Filterfunktion verworfen werden, insbesondere wobei der untere und obere Schwellwert zu physiologischen Grenzen korrespondiert, vorzugsweise wobei der untere Schwellwert einen Wert zwischen 10-50 mg/dL, insbesondere 30 mg/dL aufweist und der obere Schwellwert einen Wert zwischen 100-600 mg/dL, vorzugsweise 450 mg/dL aufweist.

11. Verfahren gemäß einem der Ansprüche 1-10, **dadurch gekennzeichnet, dass** eine Kalibrierung der Messwerte der Sensoreinrichtung nach dem Durchführen von Schritt d) erfolgt.

12. Verfahren gemäß einem der Ansprüche 1-11, **dadurch gekennzeichnet, dass** das Zustandsübergangsmodell ein Diffusionsmodell zur zeitabhängigen Modellierung des Diffusionsprozesses von Glukose von dem Transportfluid ins umgebende Gewebe umfasst

und/oder

dass Sensormodellparameter des Sensormodells und/oder Zustandsübergangsparameter des Zustandsübergangsmodells geschätzt werden, und/oder zumindest regelmäßig, aktualisiert werden.

13. Vorrichtung zum insbesondere kontinuierlichen Bestimmen eines aktuellen Glukosewerts in einem Transportfluid, insbesondere Blut, eines Organismus, vorzugsweise geeignet zur Durchführung eines Verfahrens gemäß einem der Ansprüche 1-12, umfassend

eine Sensoreinrichtung, insbesondere zur Messung von Fluoreszenz in einem das Transportfluid umgebenden Gewebe mittels seiner Glasfasersonde, ausgebildet zum Ermitteln einer Messreihe, umfassend zumindest zwei zeitlich beabstandete Messwerte, für einen Gewebe-Glukosewert in dem das Transportfluid umgebenden Gewebe,
eine Bereitstellungseinrichtung ausgebildet zum Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen, und zum Bereitstellen eines Sensormodells, wobei mittels eines Sensormodells Messwerte der Sensoreinrichtung Gewebe-Glukosewerten unter Berücksichtigung von Messrauschen zugeordnet werden,
eine Auswerteeinrichtung ausgebildet zum Ermitteln des Gewebe-Glukosewerts anhand der ermittelten Messreihe basierend auf dem Sensormodell und zum Ermitteln des aktuellen Glukosewerts basierend auf dem bereitgestellten Zustandsübergangsmodell und dem ermittelten Gewebe-Glukosewert unter Verwendung zumindest eines Moving Horizon-Schätzverfahrens.

14. Auswerteeinrichtung zum insbesondere kontinuierlichen Bestimmen eines aktuellen Glukosewerts in einem Transportfluid, insbesondere Blut, eines Organismus, vorzugsweise geeignet zur Durchführung eines Verfahrens gemäß einem der Ansprüche 1-12, umfassend

zumindest eine Schnittstelle zum Anschluss einer Sensoreinrichtung zur Bereitstellung von einer Messreihe, umfassend zumindest zwei zeitlich beabstandete Messwerte, für einen Gewebe-Glukosewert in dem das Transportfluid umgebenden Gewebe,
zumindest einen Speicher zum Speichern eines Zustandsübergangsmodells, wobei den mittels des Zustandsübergangsmodells ermittelten Gewebe-Glukosewerten zumindest ein Glukosewert in dem Transportfluid zugeordnet wird unter Berücksichtigung zumindest eines Prozessrauschwerts, und zum Speichern eines Sensormodells, wobei mittels des Sensormodells Messwerte der Sensoreinrichtung Gewebe-Glukosewerten unter Berücksichtigung von zumindest einem Messrauschwert zugeordnet werden, und

eine Recheneinrichtung ausgebildet zum Ermitteln des Gewebe-Glukosewerts anhand der ermittelten Messreihe basierend auf dem gespeicherten Sensormodell und zum Ermitteln des aktuellen Glukosewerts basierend auf dem gespeicherten Zustandsübergangsmodell und dem ermittelten Gewebe-Glukosewert unter Verwendung zumindest eines Moving Horizon-Schätzverfahrens.

15. Nichtflüchtiges computerlesbares Medium zur Speicherung von Instruktionen, welche, ausgeführt auf einem Computer, bewirken, dass ein Verfahren zum insbesondere kontinuierlichen Bestimmen eines aktuellen Glukosewerts in einem Transportfluid, insbesondere Blut, eines Organismus, durchgeführt wird, vorzugsweise geeignet zur Durchführung eines Verfahrens gemäß einem der Ansprüche 1-12,
umfassend die Schritte

a) Ermitteln, mittels einer Sensoreinrichtung, einer Messreihe, umfassend zumindest zwei zeitlich beabstandete Messwerte, für einen Gewebe-Glukosewert in dem das Transportfluid umgebenden Gewebe,
b) Ermitteln (T2) des Gewebe-Glukosewerts anhand der ermittelten Messreihe basierend auf einem Sensormodell, wobei mittels eines Sensormodells Messwerte der Sensoreinrichtung Gewebe-Glukosewerten unter Berücksichtigung von Messrauschen (V1, V2, V3, T3) zugeordnet werden,
c) Bereitstellen eines Zustandsübergangsmodells, wobei mittels des Zustandsübergangsmodells den ermittelten Gewebe-Glukosewerten zumindest ein Glukosewert in dem Transportfluid zugeordnet wird unter Berücksichtigung von Prozessrauschen (V1, V2, V3, T3), und
d) Ermitteln (S6) des aktuellen Glukosewerts basierend auf dem bereitgestellten Zustandsübergangsmodell und dem ermittelten Gewebe-Glukosewert,
e) wobei zumindest Schritt d), insbesondere die Schritte b)-d) unter Verwendung zumindest eines Moving Horizon-Schätzverfahrens durchgeführt wird.

**Claims**

1. Computer-implemented method, to preferably continuously determine a current glucose level in a transport fluid, particularly blood, of an organism, comprising the steps

a) To determine, using a sensor device, a series of measurements, comprising at least two measurements separated in time for a tissue glucose level, in the tissue surrounding the transport fluid,
b) To determine (T2) the tissue glucose level using the determined series of measurements, based on a sensor model, in which, by means of a sensor model, measurements of the sensor device are correlated to the tissue glucose levels while taking into account measurement noise (V1, V2, V3, T3),
c) To provide a state transition model, in which, by means of the state transition model, at least one glucose level in the transport fluid is correlated to the tissue glucose levels that have been determined while taking into account process noise (V1, V2, V3, T3), and
d) To provide (S6) the current glucose level based on the state transition model that has been provided and the tissue glucose level that has been determined,

**characterized in that**
at least step d), preferably steps b)-d), is carried out by using at least one Moving Horizon Estimation method, preferably wherein a Moving Horizon Estimation method is carried out to determine the current glucose level in step d) applied to previously provided glucose levels and at least one previous tissue glucose level.

2. Method according to claim 1, **characterized in that** the sensor model is provided in the form of a linear function or a non-linear function between measurements and tissue glucose levels.

3. Method according to one of claims 1-2, **characterized in that** the value for the horizon of the Moving Horizon Estimation method to determine the current glucose level is selected as less than or equal to 10.

4. Method according to one of claims 1-3, **characterized in that** a variance of the measurement noise and/or the variance of the process noise, particularly at least on a regular basis, is estimated, preferably wherein the variance of the measurement noise and/or the variance of the process noise is estimated on the basis of at least one previous respective value, in particular interpolated and/or weighted, preferably using an exponential smoothing.

5. Method according to claim 4, **characterized in that** values that are only partially required to calculate the estimation of the measurement noise and/or of the process noise are temporarily stored, and values that have not been temporarily stored and that are needed are interpolated using the values that have been stored.

6. Method according to claims 3 and 4, **characterized in that** a number of the previous respective values, is selected to be greater than the value for the horizon of the Moving Horizon Estimation method, in particular at least twice as great, preferably at least by the factor 5.

7. Method according to claims 4 and 6, **characterized in that** the variance of the measurement noise and/or the variance of the process noise is regularly adjusted based on the value of the sum of the horizon of the Moving Horizon Estimation method and the number of the previous respective values to calculate the estimation of the measurement noise and/or the process noise.

8. Method according to one of claims 1-7, **characterized in that** determined values are filtered by means of a filter function, whereby, by means of the filter function, errors, especially measurement errors, of the sensor device, are suppressed, preferably wherein measurement noise values are weighted by means of the filter function.

9. Method according to claim 8, **characterized in that** in order to determine errors of the sensor device, the gradient of the increase in a current tissue glucose level and/or the current tissue glucose level is evaluated.

10. Method according to one of claims 8 or 9, **characterized in that** determined measurements being below a low threshold level that can be preset and/or above a high threshold level that can be preset are rejected by means of the filter function, particularly wherein the low and high threshold level corresponds to physiological limits, preferably where the low threshold level presents a value between 10-50 mg/dL, particularly 30 mg/dL and the high threshold level presents a value between 100-600 mg/dL, preferably 450 mg/dL.

11. Method according to one of claims 1-10, **characterized in that** a calibration of the measurements of the sensor device after step d) is carried out.

12. Method according to one of claims 1-11, **characterized in that** the state transition model comprises a diffusion model for time-dependent modelling of the diffusion process of glucose from the transport fluid into the surrounding tissue,
and/or that
sensor model parameters of the sensor model and/or state transition parameters of the state transition model are estimated and/or updated, at least on a regular basis.

13. Device to preferably continuously determine a current glucose level in a transport fluid, particularly blood, of an organism, preferably suitable for carrying out a method according to one of claims 1-12, comprising

   a sensor device, particularly for measuring fluorescence in a tissue surrounding the transport fluid, by means of a fiber optic probe, designed to determine a series of measurements, comprising at least two measurements separated in time for a tissue glucose level in the tissue surrounding the transport fluid,
   a provision device designed to provide a state transition model, in which, by means of the state transition model, at least one glucose level in the transport fluid is correlated to the tissue glucose levels that have been determined while taking into account process noise, and to provide a sensor model, in which, by means of a sensor model, measurements of the sensor device are correlated to tissue glucose levels taking into account measurement noise,
   an evaluating device designed to determine the tissue glucose level, using the series of determined measurements, based on the sensor model and to determine the current glucose level based on the state transition model that has been provided and the tissue glucose level that has been determined, using at least one Moving Horizon Estimation method.

14. Evaluation device to preferably continuously determine a current glucose level in a transport fluid, particularly blood, of an organism, preferably suitable for carrying out a method according to one of claims 1-12, comprising at least one interface to connect a sensor device to provide a series of measurements, comprising at least two measurements separated in time for a tissue glucose level in the tissue surrounding the transport fluid,

   at least one memory to store a state transition model, in which, at least one glucose level in the transport fluid is correlated to the tissue glucose levels determined by the state transition model, while taking into account at least one process noise value, and to store a sensor model, in which, by means of the sensor model, measurements of the sensor device are correlated to tissue glucose levels while taking into account at least one measurement noise value, and

a calculating device designed to determine the tissue glucose level, using the determined series of measurements, based on the stored sensor model and to determine the current glucose level based on the state transition model that has been stored and the tissue glucose level that has been determined, using at least one Moving Horizon Estimation method.

15. Non-tangible, computer-readable medium for storing instructions, which, when carried out on a computer, cause a method to preferably continuously determine a current glucose level in a transport fluid, particularly blood, of an organism, particularly suitable for performing a method according to one of the claims 1-12, comprising the steps

a) To determine, using a sensor device, a series of measurements, comprising at least two measurements separated in time for a tissue glucose level, in the tissue surrounding the transport fluid,
b) To determine (T2) the tissue glucose level using the determined series of measurements, based on a sensor model, in which, by means of a sensor model, measurements of the sensor device are correlated to the tissue glucose levels while taking into account measurement noise (V1, V2, V3, T3),
c) To provide a state transition model, in which, by means of the state transition model, at least one glucose level in the transport fluid is correlated to the tissue glucose levels that have been determined while taking into account process noise (V1, V2, V3, T3), and
d) To provide (S6) the current glucose level based on the state transition model that has been provided and the tissue glucose level that has been determined,
e) wherein at least step d), preferably steps b)-d), is carried out by using at least one Moving Horizon Estimation method.

**Revendications**

1. Procédé implémenté par ordinateur pour la détermination, plus particulièrement continue, d'une valeur de glucose actuelle dans un fluide de transport, plus particulièrement du sang, d'un organisme, comprenant les étapes suivantes :

a) détermination, au moyen d'un dispositif de capteur, d'une série de mesures, comprenant au moins deux valeurs de mesure espacées dans le temps, pour une valeur de glucose de tissu, dans le tissu entourant le fluide de transport,
b) détermination (T2) de la valeur de glucose du tissu à l'aide de la série de mesures déterminée sur la base d'un modèle de capteur, dans lequel, au moyen d'un modèle de capteur, les valeurs de mesure du dispositif de capteur sont attribuées aux valeurs de glucose du tissu en tenant compte du bruit des mesures (V1, V2, V3, T3),
c) mise à disposition d'un modèle de transition d'état, dans lequel, au moyen du modèle de transition d'état, aux valeurs de glucose du tissu déterminées est attribuée au moins une valeur de glucose dans le fluide de transport en tenant compte du bruit du processus (V1, V2, V3, T3) et
d) détermination (S6) de la valeur actuelle du glucose sur la base du modèle de transition d'état mis à disposition et de la valeur de glucose du tissu déterminée,

**caractérisé en ce que**
au moins l'étape d), plus particulièrement les étapes b) - d), est exécutée à l'aide d'au moins un procédé d'estimation de l'horizon mobile, de préférence dans lequel un procédé d'estimation de l'horizon mobile est appliqué, pour la détermination de la valeur de glucose actuelle à l'étape d), à des valeurs de glucose déterminées auparavant et au moins à une valeur de glucose de tissu précédente.

2. Procédé selon la revendication 1, **caractérisé en ce que** le modèle de capteur est mis à disposition sous la forme d'une fonction linéaire ou d'une fonction non linéaire entre les valeurs de mesure et les valeurs de glucose du tissu.

3. Procédé selon l'une des revendications 1 à 2, **caractérisé en ce que** la valeur choisie pour l'horizon du procédé d'estimation de l'horizon mobile pour la détermination de la valeur de glucose actuelle est inférieure ou égale à 10.

4. Procédé selon l'une des revendications 1 à 3, **caractérisé en ce qu'**une variance du bruit de mesure et/ou une variance du bruit de processus, sont estimés, plus particulièrement au moins régulièrement, de préférence dans lequel la variance du bruit de mesure et/ou la variance du bruit de processus est estimée sur la base d'au moins une valeur respective précédente, plus particulièrement interpolée et/ou pondérée, de préférence à l'aide d'un lissage exponentiel.

5. Procédé selon la revendication 4, **caractérisé en ce que**, seules des valeurs partiellement nécessaires pour le calcul de l'estimation du bruit de mesure et/ou du bruit de processus sont enregistrées et les valeurs non enregistrées nécessaires sont interpolées à l'aide des valeurs enregistrées.

6. Procédé selon la revendication 3 et 4, **caractérisé en ce qu'**un nombre choisi de valeurs respectives précédentes est supérieur à la valeur pour l'horizon du procédé d'estimation de l'horizon mobile, plus particulièrement est au moins le double, de préférence multiplié au moins par le facteur 5.

7. Procédé selon la revendication 4 et 6, **caractérisé en ce que** la variance du bruit de mesure et/ou la variance du bruit de processus est adapté régulièrement sur la base de la valeur de la somme de l'horizon du procédé d'estimation de l'horizon mobile et du nombre de valeurs respectives précédentes pour le calcul de l'estimation du bruit de mesure et/ou du bruit de processus.

8. Procédé selon l'une des revendications 1 à 7, **caractérisé en ce que** les valeurs déterminées sont filtrées au moyen d'une fonction de filtrage, dans lequel, au moyen de la fonction de filtrage, les erreurs, plus particulièrement les erreurs de mesure, du dispositif de capteur, sont atténuées, de préférence dans lequel les valeurs du bruit de mesure sont pondérées au moyen de la fonction de filtrage.

9. Procédé selon la revendication 8, **caractérisé en ce que**, pour la détection des erreurs du dispositif de capteur, le gradient de l'augmentation dans une valeur de glucose de tissu actuelle et/ou la valeur de glucose de tissu actuelle sont analysés.

10. Procédé selon l'une des revendications 8 ou 9, **caractérisé en ce que** les valeurs de mesure déterminées en dessous d'une valeur seuil inférieure prédéterminée et/ou au-dessus d'une valeur supérieure prédéterminée sont rejetées au moyen de la fonction de filtrage, plus particulièrement dans lequel la valeur seuil inférieure et supérieure correspond à des limites physiques, de préférence dans lequel la valeur de seuil inférieure présente une valeur entre 10-50 mg/dL, plus particulièrement 30 mg/dL et la valeur seuil supérieure présente une valeur entre 100-600 mg/dL, de préférence 450 mg/dL.

11. Procédé selon l'une des revendications 1 à 10, **caractérisé en ce qu'**un calibrage des valeurs d mesure du dispositif de capteur a lieu avec l'exécution de l'étape d).

12. Procédé selon l'une des revendications 1 à 11, **caractérisé en ce que** le modèle de transition d'état comprend un modèle de diffusion pour la modélisation indépendante du temps du processus de diffusion du glucose du fluide de transport vers le tissu environnant,
et/ou
les paramètres de modèle de capteur du modèle capteur et/ou les paramètres de transition d'état du modèle de transition d'état sont estimés et/ou actualisés au moins régulièrement.

13. Dispositif pour la détermination, plus particulièrement continue, d'une valeur de glucose actuelle dans un fluide de transport, plus particulièrement du sang, d'un organisme, de préférence conçu pour l'exécution d'un procédé selon l'une des revendications 1 à 12, comprenant

un dispositif de capteur, plus particulièrement pour la mesure de fluorescence dans un tissu entourant le fluide de transport au moyen d'une sonde à fibre de verre, conçu pour la détermination d'une série de mesures, comprenant au moins deux valeurs de mesure espacées dans le temps, pour une valeur de glucose de tissu dans le tissu entourant le fluide de transport,
un dispositif de mise à disposition conçu pour la mise à disposition d'un modèle de transition d'état, dans lequel, au moyen du modèle de transition d'état, aux valeurs de glucose du tissu déterminées, est attribuée au moins une valeur de glucose dans le fluide de transport en tenant compte du bruits du processus et, pour la mise à disposition d'un modèle de capteur, dans lequel au moyen d'un modèle de capteur, les valeurs de mesure du dispositif de capteur sont attribuées à des valeurs de glucose du tissu en tenant compte du bruits des mesures,
un dispositif d'analyse conçu pour la détermination de la valeur de glucose du tissu à l'aide de la série de mesures déterminées sur la base du modèle de capteur et pour la détermination de la valeur de glucose actuelle sur la base du modèle de transition d'état mis à disposition et de la valeur de glucose du tissu déterminée à l'aide d'au moins un procédé d'estimation d'horizon mobile.

**14.** Dispositif d'analyse pour la détermination, plus particulièrement continue, d'une valeur de glucose actuelle dans un fluide de transport, plus particulièrement du sang, d'un organisme, de préférence conçu pour l'exécution d'un procédé selon l'une des revendications 1 à 12, comprenant

au moins une interface pour le raccordement d'un dispositif de capteur pour la mise à disposition d'une série de mesures, comprenant au moins deux valeurs de mesure espacées dans le temps, pour une valeur de glucose de tissu dans le tissu entourant le fluide de transport,
au moins une mémoire pour l'enregistrement d'un modèle de transition d'état, dans lequel, aux valeurs de glucose du tissu, déterminées au moyen du modèle de transition d'état, est attribuée au moins une valeur de glucose dans le fluide de transport en tenant compte d'au moins une valeur de bruit de processus, et pour l'enregistrement d'un modèle de capteur, dans lequel, au moyen du modèle de capteur, les valeurs de mesure du dispositif de capteur sont attribuées à des valeurs de glucose de tissus en tenant compte d'au moins une valeur de bruit de mesure et
un dispositif de calcul conçu pour la détermination de la valeur de glucose de tissu à l'aide de la série de mesures déterminées sur la base du modèle de capteur enregistré et pour la détermination de la valeur de glucose actuelle sur la base du modèle de transition d'état enregistré et de la valeur de glucose de tissu déterminée à l'aide d'au moins un procédé d'estimation d'horizon mobile.

**15.** Support non volatil lisible par un ordinateur, pour le stockage d'instructions qui, exécutées sur un ordinateur, font en sorte qu'un procédé de détermination, plus particulièrement continue, d'une valeur de glucose actuelle dans un fluide de transport, plus particulièrement du sang, d'un organisme, soit exécuté, de préférence conçu pour l'exécution d'un procédé selon l'une des revendications 1 à 12,
comprenant les étapes suivantes :

a) détermination, au moyen d'un dispositif de capteur, d'une série de mesures, comprenant au moins deux valeurs de mesure espacées dans le temps, pour une valeur de glucose de tissu dans le tissu entourant le fluide de transport,
b) détermination (T2) de la valeur de glucose du tissu à l'aide de la série de mesures déterminée sur la base d'un modèle de capteur, dans lequel, au moyen d'un modèle de capteur, les valeurs de mesure du dispositif de capteur sont attribuées aux valeurs de glucose du tissu en tenant compte du bruit des mesures (V1, V2, V3, T3),
c) mise à disposition d'un modèle de transition d'état, dans lequel, au moyen du modèle de transition d'état, aux valeurs de glucose du tissu déterminées est attribuée au moins une valeur de glucose dans le fluide de transport en tenant compte du bruit du processus (V1, V2, V3, T3) et
d) détermination (S6) de la valeur actuelle du glucose sur la base du modèle de transition d'état mis à disposition et de la valeur de glucose du tissu déterminée,

dans lequel
au moins l'étape d), plus particulièrement les étapes b) - d), est exécutée à l'aide d'au moins un procédé d'estimation de l'horizon mobile.

Fig. 1

a)

b)

c)

d)

Fig. 2

Fig. 3

EP 3 849 419 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006017358 A1
- DE 102015101847 B4
- US 2011237917 A1
- US 2013079613 A1


**In der Beschreibung aufgeführte Nicht-Patentliteratur**

- **BASU, ANANDA et al.** Time lag of Glucose from intravascular to interstitial compartment in humans. *Diabetes,* 2013, 131132
- **REBRIN, KERSTIN et al.** Subcutaneous Glucose predicts plasma Glucose independent of insulin: implications for continuous monitoring. *American Journal of Physiology-Endocrinology and Metabolism,* 1999, vol. 277 (3), E561-E571
- **KEENAN, D. BARRY et al.** Delays in minimally invasive continuous Glucose monitoring devices: a review of current technology. *Journal of diabetes science and technology,* 2009, vol. 3 (5), 1207-1214
- **KNOBBE, EDWARD J. ; BRUCE BUCKINGHAM.** The extended Kalman filter for continuous Glukose monitoring. *Diabetes technology & therapeutics,* 2005, vol. 7 (1), 15-27
- **GRACE WAHBA.** Bayesian ''Confidence Intervals'' for the Cross-Validated Smoothing Spline. *Journal oft he Royal Statistical Society: Series B (Methodological),* 1983, vol. 45, 133-150
- **RIOS ; SAHINIDIS.** Derivative-free optimization: a review of algorithms and comparison of software implementations. *Journal of Global Optimization,* 2013, vol. 56 (3), 1247-1293
- **KÜSTER, NIKOLAUS et al.** First Clinical Evaluation of a New Percutaneous Optical Fiber Glukose Sensor for Continuous Glukose Monitoring in Diabetes. *Journal of Diabetes Science and Technology,* 2014, vol. 7 (1), 13-23